# EUROPEAN PATENT APPLICATION

(11) **EP 1 598 355 A1**
(43) Date of publication of application: **23.11.2005**
(21) Application number: 04700527.7
(22) Date of filing: 07.01.2004
(51) Int. Cl.: C07D 491/048, A61K 31/4741, A61P 25/18, A61P 25/28, A61P 43/00

(54) **THERAPEUTIC AGENT FOR SCHIZOPHRENIA**

(30) Priority: 08.01.2003 JP 2003001817
(71) Applicant: Mitsubishi Pharma Corporation, Osaka-shi, Osaka 541-0046 (JP)
(72) Inventor: BESSHO, Tomoko, c/o MITSUBISHI PHARMA CORPORATION, Chuo-ku, Tokyo 1038405 (JP); TAKASHINA, Ken, c/o MITSUBISHI PHARMA CORPORATION, Chuo-ku, Tokyo 1038405 (JP)
(74) Representative: HOFFMANN EITLE
(86) International application number: PCT/JP2004/000023
(87) International publication number: WO 2004/063201

(57) **Abstract**

Provision of a pharmaceutical agent useful for the treatment of schizophrenia. As a solving means, a therapeutic agent for schizophrenia containing a 4-acylamino-5,6,7,8-tetrahydrofuro[2,3-b]quinoline derivative, an enantiomer thereof, an acid addition salt thereof or a hydrate or solvate thereof as an active ingredient is provided.

## Description

### Technical Field

The present invention relates to a therapeutic agent for schizophrenia, which comprises a 4-acylaminotetrahydrofuro[2,3-b]quinoline derivative, an enantiomer thereof, an acid addition salt thereof or a hydrate or solvate thereof as an active ingredient.

### Background Art

Schizophrenia is a disease of psychological disorder that is developed in a little less than 1% of general population, and the symptoms thereof are classified into positive symptoms such as psychomotor excitation, hallucination, delusion and the like, negative symptoms such as aspontaneity, apathia, flexibility disorder and the like and cognitive disorders. Heretofore, therapeutic drugs for schizophrenia have been developed with positive symptoms as the target condition. However, negative symptoms and cognitive disorders are deeply involved in the chronicity of schizophrenia and the difficulty in rehabilitation (Non-patent Reference 1), and a pharmaceutical agent having an improving action of such conditions has been strongly desired. Thus, improving not only positive symptoms but also negative symptoms and cognitive disorders is considered to lead to a useful therapeutic method for schizophrenia. In these several years, pharmaceutical agents showing an improving action on not only positive symptoms but also negative symptoms and cognitive disorders have been developed (Non-patent Reference 2), but their number is still small and the effects are not sufficient.

Meanwhile, Phencyclidine (PCP) was developed as a dissociative anesthetic but its clinical use was relinquished because it causes schizophrenia-like symptoms during decubation from anesthesia (Non-patent Reference 3). PCP is known to express not only positive symptoms but also negative symptoms and cognitive disorders (Non-patent Reference 4; Non-patent Reference 5). In search of a therapeutic drug for not only positive symptoms but also negative symptoms and cognitive disorders in schizophrenia, a method of studying an action on changes in the behavior induced by the administration of PCP to animals has been used as a schizophrenia model.

A 4-acylaminotetrahydrofuro[2,3-b]quinoline derivative is known as a pharmaceutical agent that improves lowered high affinity choline uptake ability, activates cholinergic nerves, and improves memory disturbance such as Alzheimer's disease and the like (Patent Reference 1), but its effect on a model of schizophrenia among the diseases of psychological disorder has not been known. There is a report that Donepezil, which is known as a pharmaceutical agent that activates cholinergic nerves by a different mechanism of inhibition of degradation of acetylcholine, and improves memory disturbances such as Alzheimer's disease and the like, improved cognitive disorders in schizophrenia (Non-patent Reference 6), but its effect is still uncertain.
(Patent Reference 1)
JP-A-3-218361
(Non-patent Reference 1)
Rinsho Seishin Yakuri 5: 1249-1256, 2002
(Non-patent Reference 2)
Rinsho Seishin Yakuri 5: 167-176, 2002
(Non-patent Reference 3)
NIDA Res Monogr 64: 148-162, 1986
(Non-patent Reference 4)
Am J Psychiatry 148: 1301-1308, 1991
(Non-patent Reference 5)
Semin Nucl Med 22: 254-267, 1992
(Non-patent Reference 6)
Schizophrenia Research 59: 29-33, 2002

### Disclosure of the Invention

The present inventors have conducted intensive studies in an attempt to develop a pharmaceutical agent useful for schizophrenia, and first found that a 4-acylaminotetrahydrofuro[2,3-b]quinoline derivative is effective for a schizophrenia model, and completed the present invention.

Accordingly, the present invention provides the following.
[1] A therapeutic agent for schizophrenia, which comprises, as an active ingredient, a compound of the formula (I) 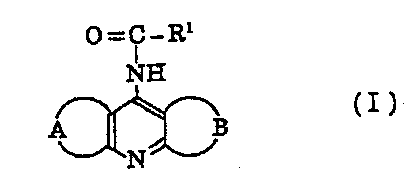 wherein R¹ is a C₂-C₆ alkyl group or the formula (II) 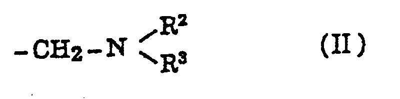 wherein R² is a hydrogen atom or an acetyl group and R³ is a C₁-C₆ alkyl group, a cycloalkyl group or 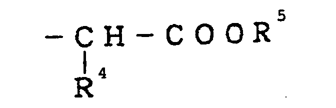 wherein R⁴ and R⁵ are each independently a hydrogen atom or a C₁-C₆ alkyl group, and in 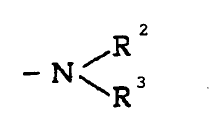 of the formula (II) , R² and R³ may be linked to each other to form 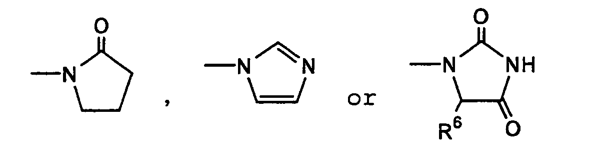 wherein R⁶ is a hydrogen atom or a C₁-C₆ alkyl group; 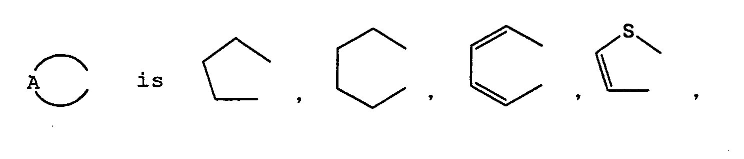 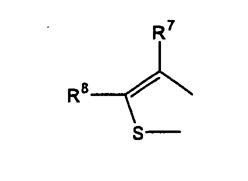 wherein R⁷ and R⁸ are each independently a hydrogen atom or a C₁-C₄ alkyl group, 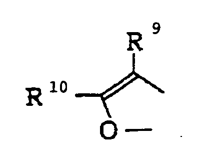 wherein R⁹ and R¹⁰ are each independently a hydrogen atom or a C₁-C₄ alkyl group, 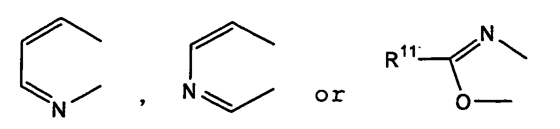 wherein R¹¹ is a hydrogen atom or a C₁-C₄ alkyl group; and 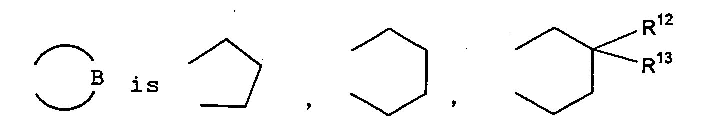 wherein R¹² and R¹³ are each independently a C₁-C₄ alkyl group or may be linked to each other to form 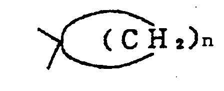 wherein n is an integer of 2 to 6, or 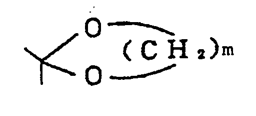 wherein m is an integer of 2 or 3, 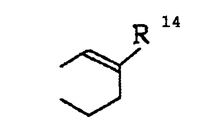 wherein R¹⁴ is a hydrogen atom or a C₁-C₄ alkyl group, 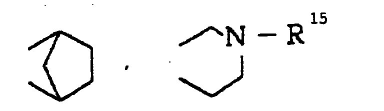 wherein R¹⁵ is a hydrogen atom or an aralkyl group, or 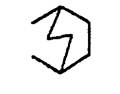 provided that when 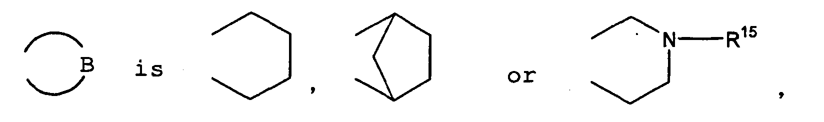 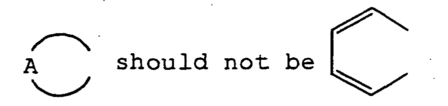 and R⁷ should not be a hydrogen atom, an enantiomer thereof, an acid addition salt thereof, or a hydrate or solvate thereof.
[2] The pharmaceutical agent of the above-mentioned [1], wherein the compound of the formula (I) is a compound of the formula (Ia) 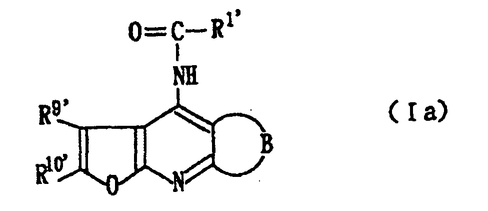 wherein R^{1'} is a C₂-C₆ alkyl group or the formula (II)' 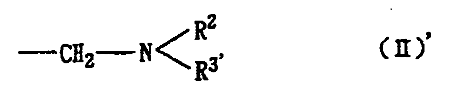 wherein R² is a hydrogen atom or an acetyl group, and R^{3'} is a C₁-C₆ alkyl group or 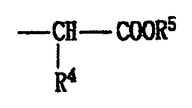 wherein R⁴ and R⁵ are each independently a hydrogen atom or a C₁-C₆ alkyl group, and in 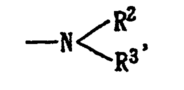 of the formula (II)', R² and R^{3'} may be linked to each other to form 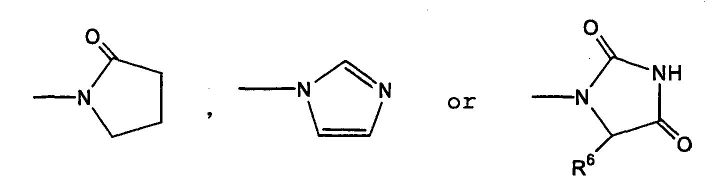 wherein R⁶ is a hydrogen atom or a C₁-C₆ alkyl group; R^{9'} and R^{10'} are each independently a C₁-C₄ alkyl group; and 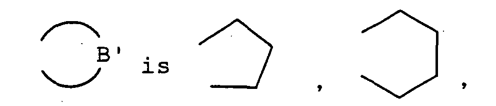 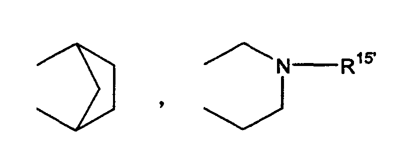 wherein R^{15'} is an aralkyl group, or 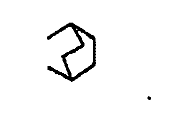
[3] The pharmaceutical agent of the above-mentioned [1], wherein the compound of the formula (I) is a 4-acylamino-5,6,7,8-tetrahydrofuro[2,3-b]quinoline derivative of the formula (Ib) 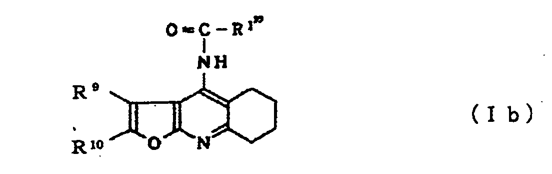 wherein R^{1"} is a C₂-C₆ alkyl group, 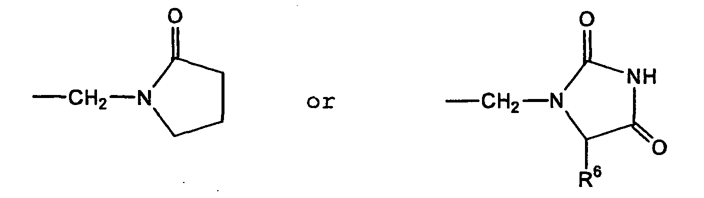 wherein R⁶ is a hydrogen atom or a C₁-C₆ alkyl group; and R⁹ and R¹⁰ are each independently a hydrogen atom or a C₁-C₄ alkyl group.
[4] The pharmaceutical agent of the above-mentioned [1], wherein the compound of the formula (I) is 2-(2-oxopyrrolidin-1-yl)-N-(2,3-dimethyl-5,6,7,8-tetrahydrofuro[2,3-b]quinolin-4-yl)acetamide.
[5] The therapeutic agent for schizophrenia of any of the above-mentioned [1]-[4], wherein the condition of schizophrenia is a negative symptom or a cognitive disorder.

In a preferable embodiment of the present invention, the invention is a therapeutic agent for schizophrenia, particularly preferably for a negative symptom or a cognitive disorder.

### Brief Description of the Drawings

### Fig. 1

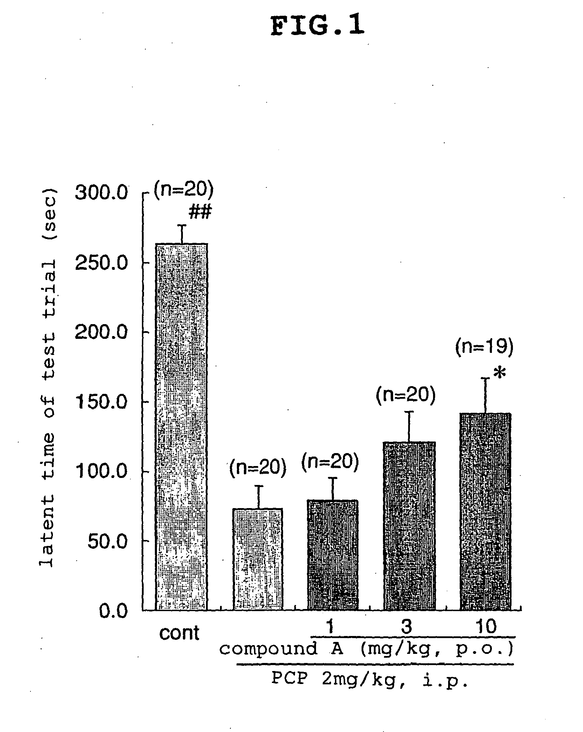

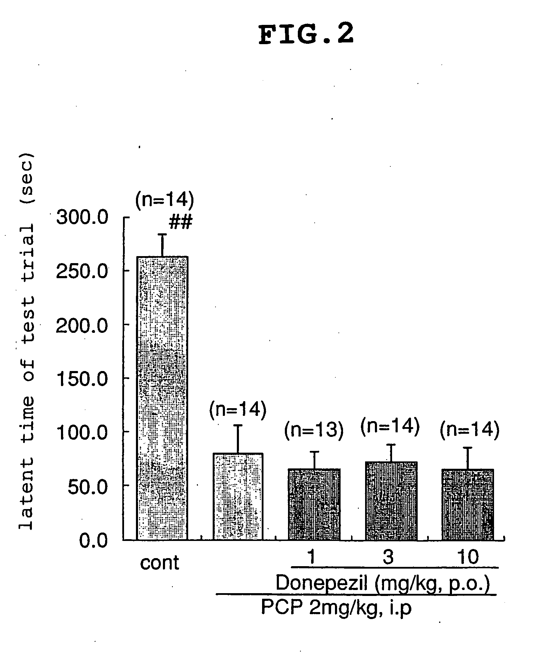

Fig. 1 shows the action of compound A on a Phencyclidine (PCP)-induced passive avoidance reaction disorder, wherein the horizontal axis shows a drug administration group (1, 3, 10 mg/kg of administration) and the vertical axis shows a latent time (sec) of test trial.

### Fig. 2

A reference Figure that shows an action of Donepezil on a Phencyclidine (PCP)-induced passive avoidance reaction disorder, wherein the horizontal axis shows a drug administration group (1, 3, 10 mg/kg of administration) and the vertical axis shows a latent time (sec) of test trial.

### Best Mode for Embodying the Invention

The therapeutic agent for schizophrenia of the present invention contains a pyrazolone derivative represented by the formula (I) defined in the present specification, or a physiologically acceptable salt, or a hydrate or solvate thereof.

As the C₂-C₆ alkyl group to be used in the present invention for R¹, R^{1'} or R^{1"}, C₂-C₄ alkyl groups such as ethyl group, n-propyl group, isopropyl group, n-butyl group, sec-butyl group, tert-butyl group and the like can be preferably mentioned.

As the C₁-C₆ alkyl group for R³ or R^{3'}, C₁-C₄ alkyl groups such as methyl group, ethyl group, n-propyl group, isopropyl group, n-butyl group, sec-butyl group, tert-butyl group and the like can be preferably mentioned. As the cycloalkyl group for R³, C₃-C₆ cycloalkyl groups such as cyclopropyl group, cyclobutyl group, cyclopentyl group, cyclohexyl group and the like can be preferably mentioned.

As the C₁-C₆ alkyl group for R⁴-R⁶, C₁-C₄ alkyl groups such as methyl group, ethyl group, n-propyl group, isopropyl group, n-butyl group, sec-butyl group, tert-butyl group and the like can be preferably mentioned.

As the C₁-C₄ alkyl group for R⁷-R¹⁴, R^{9'} or R^{10'}, C₁-C₄ alkyl groups such as methyl group, ethyl group, n-propyl group, isopropyl group, n-butyl group, sec-butyl group, tert-butyl group and the like can be preferably mentioned.

As the aralkyl group for R¹⁵ or R^{15'}, phenyl groups substituted by C₁-C₄ alkyl group, such as benzyl group, phenethyl group and the like can be mentioned.

As the acid for the acid addition salt of the compound represented by the formula (I) in the present invention (encompassing compounds represented by the formula (Ia) and the formula (Ib), hereinafter these compounds are also generally referred to as the compound of the formula (I) for convenience), inorganic acids such as hydrochloric acid, hydrobromic acid, hydroiodic acid, sulfuric acid, phosphoric acid and the like, and organic acids such as oxalic acid, maleic acid, fumaric acid, lactic acid, malic acid, citric acid, tartaric acid, benzoic acid, methanesulfonic acid, camphor sulfonic acid and the like can be mentioned. The acid addition salts that can be administered are those acceptable as a pharmaceutical agent.

The above-mentioned compound of the formula (I) and acid addition salt thereof may be present in the form of a hydrate or a solvate, and therefore, these hydrates and solvates are encompassed in the compound to be the active ingredient of the present invention. In addition, the above-mentioned compound of the formula (I) sometimes has an enantiomer, and the enantiomer is also encompassed in the compound to be the active ingredient of the present invention. The production method of the compound of the formula (I) and the like contained in the drug or pharmaceutical agent of the present invention as an active ingredient is not particularly limited, and can be easily synthesized suitably by, for example, a method described in JP-A-3-218361 (patent No. 2546919) or by a method known in the art.

The above-mentioned compound of the formula (I), particularly the compound of the formula (Ia), particularly preferably 4-acylamino-5,6,7,8-tetrahydrofuro[2,3-b]quinoline derivative, which is the compound of the formula (Ib), and the like showed an action of improving the disorder of passive avoidance reaction in Phencyclidine (PCP)-induced passive avoidance reaction disorder in rats, which is a model of changes in the behavior of cognitive disorder and the like in schizophrenia, as shown in the Example below. By this, it has become possible to improve changes in the behavior of cognitive disorder and the like in schizophrenia and treat schizophrenia. A 4-acylamino-5,6,7,8-tetrahydrofuro[2,3-b]quinoline derivative of the above-mentioned formula (Ib) and the like having such action can be used as a therapeutic drug for schizophrenia.

While the dose of the pharmaceutical agent of the present invention is not particularly limited, it is generally 1-2000 mg/kg body weight/day, preferably 1-500 mg/kg body weight/day, for oral administration and 0.1-100 mg/kg body weight/day, preferably 0.1-50 mg/kg body weight/day, for parenteral administration, both in the weight of the compound of the formula (I), which is the active ingredient. The above-mentioned dose is preferably administered once a day or in 2-3 portions a day, which may be appropriately increased or decreased according to the age, disease state and condition.

As the pharmaceutical agent of the present invention, the above-mentioned compound represented by the formula (I) or a physiologically acceptable salt thereof, or a hydrate or solvate thereof may be administered as it is. Generally, however, it is preferable to prepare and administer a pharmaceutical composition containing the above-mentioned substance, which is the active ingredient, and a pharmacologically and pharmaceutically acceptable additive.

As the pharmacologically and pharmaceutically acceptable additive, for example, an excipient, a disintegrant or a disintegration aid, a binder, a lubricant, a coating agent, a pigment, a diluent, a base, a solubilizer or a dissolution aid, an isotonic agent, a pH regulator, a stabilizer, a propellant, an adhesive and the like can be used.

For a pharmaceutical composition suitable for oral administration, for example, excipients such as glucose, lactose, D-mannitol, starch, crystalline cellulose and the like; disintegrants or disintegration aids such as carboxymethyl cellulose, starch, carboxymethylcellulose calcium and the like; binders such as hydroxypropylcellulose, hydroxypropylmethylcellulose, polyvinylpyrrolidone, gelatin and the like; lubricants such as magnesium stearate, talc and the like; coating agents such as hydroxypropylmethylcellulose, sucrose, polyethylene glycol, titanium oxide and the like; and bases such as petrolatum, liquid paraffin, polyethylene glycol, gelatin, kaolin, glycerin, purified water, hard fat and the like can be used as an additive.

For a pharmaceutical composition suitable for injection or infusion, additives such as solubilizers or dissolution aids capable of constituting an aqueous or dissolution in use type injection (e.g., distilled water for injection, physiological saline, propylene glycol and the like); isotonic agents (e.g., glucose, sodium chloride, D-mannitol, glycerin and the like); pH regulators (e.g., inorganic acid, organic acid, inorganic base, organic base and the like); and the like can be used.

The form of the pharmaceutical agent of the present invention is not particularly limited and can take various forms capable of being used by those of ordinary skill in the art. As a pharmaceutical agent suitable for oral administration, for example, tablet, powder, granule, hard gelatin capsule, suppository, troche and the like can be prepared using an additive for solid preparation, and syrup, emulsion, soft gelatin capsule and the like can be prepared using an additive for liquid preparation. In addition, as a pharmaceutical agent suitable for parenteral administration, injection, drop, inhalant, suppository, percutaneous absorber, per mucosa absorber and the like can be prepared.

The pharmaceutical agent of the present invention is effective for therapy of schizophrenia. In other words, the pharmaceutical agent of the present invention has an action of a therapeutic agent that cures schizophrenia into a normal state.

In the present specification, "schizophrenia" is interpreted in a widest sense. To be precise, "schizophrenia" in the context of the present invention includes all major psychotic syndromes: (1) schizophreniform, (2) schizophreniform disorder, (3) delusional disorder, (4) brief psychotic disorder and the like.

The administration route of the pharmaceutical agent of the present invention is not particularly limited and the agent can be administered orally or parenterally.

### Example

The present invention is explained in more detail by referring to the following Example, which is not to be construed as limitative.

### Example 1: Action on Phencyclidine (PCP)-induced passive avoidance reaction disorder in rats

For the test, twenty 8-week-old Wistar male rats were used per group.

To the rats was orally administered 1, 3, 10 mg/kg (body weight) of 2-(2-oxopyrrolidin-1-yl)-N-(2,3-dimethyl-5,6,7,8-tetrahydrofuro[2,3-b]quinolin-4-yl)acetamide (hereinafter compound A) suspended in 0.5% Tween 80 solution. To the control group and vehicle group was orally administered an equivalent amount of 0.5% Tween 80 solution. At 30 min after oral administration, PCP dissolved in physiological saline was intraperitoneally administered at 2 mg/kg (body weight). To the control group was intraperitoneally administered an equivalent amount of physiological saline. At 30 min after PCP or physiological saline administration, acquisition trial of passive avoidance reaction was performed. In the acquisition trial, the rats were placed in a light room (50 cm x 50 cm x 50 cm) and, when the rats moved to a dark room (20 cm x 14 cm x 20 cm), a guillotine door between the light room and the dark room was closed and footshock was loaded for 5 sec from the floor grid in the dark room.

In a test trial, at 24 hr after the acquisition trial, the rats were placed in the light room again and the latent time until move to the dark room was measured for the maximum of 300 seconds. When the rats did not move to the dark room within 300 seconds, the latent time was considered to be 300 seconds.

The results are shown in Fig. 1. In the vehicle group, the latent time during the test was significantly shortened by PCP administration as compared to the control group, and disorder in the passive avoidance reaction was observed. At 10 mg/kg, compound A significantly prolonged the latent time shortened by the PCP administration, and showed an action of improving disorder in the passive avoidance reaction.

### Industrial Applicability

The pharmaceutical agent of the present invention is useful for the treatment of schizophrenia. Particularly, the pharmaceutical agent of the present invention shows an effect of improving disorder in the passive avoidance reaction by PCP, which is a schizophrenia model including negative symptoms and cognitive disorders. Therefore, the agent is clinically useful for the negative symptoms and cognitive disorders thereof.

This application is based on a patent application No. 001817/2003 filed in Japan.

## Claims

1. A therapeutic agent for schizophrenia, which comprises, as an active ingredient, a compound of the formula (I) 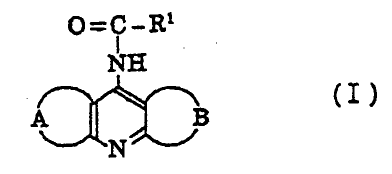 wherein R¹ is a C₂-C₆ alkyl group or the formula (II) 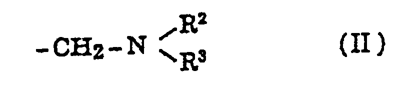 wherein R² is a hydrogen atom or an acetyl group and R³ is a C₁-C₆ alkyl group, a cycloalkyl group or 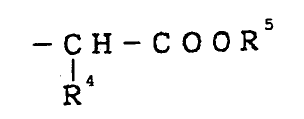 wherein R⁴ and R⁵ are each independently a hydrogen atom or a C₁-C₆ alkyl group, and in 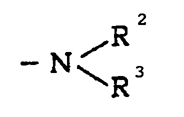 of the formula (II) , R² and R³ may be linked to each other to form 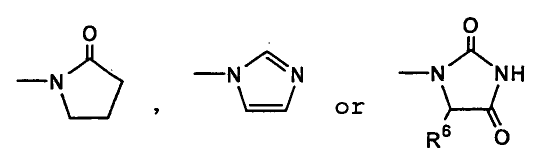 wherein R⁶ is a hydrogen atom or a C₁-C₆ alkyl group; 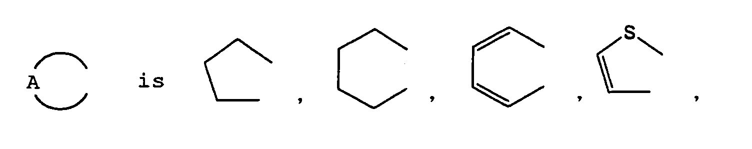 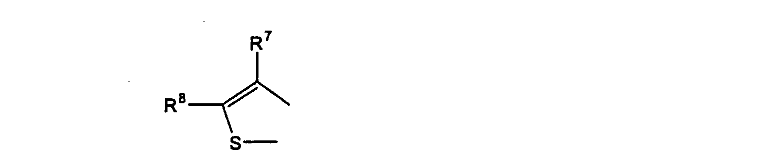 wherein R⁷ and R⁸ are each independently a hydrogen atom or a C₁-C₄ alkyl group, 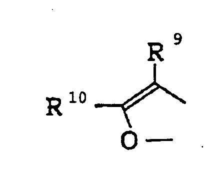 wherein R⁹ and R¹⁰ are each independently a hydrogen atom or a C₁-C₄ alkyl group, 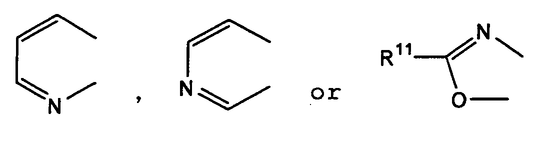 wherein R¹¹ is a hydrogen atom or a C₁-C₄ alkyl group; and 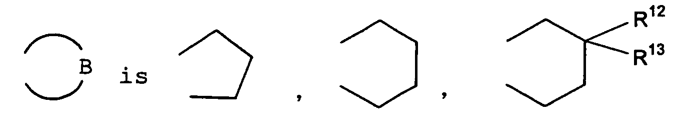 wherein R¹² and R¹³ are each independently a C₁-C₄ alkyl group or may be linked to each other to form 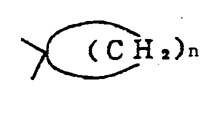 wherein n is an integer of 2 to 6, or 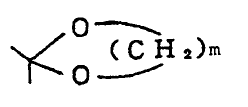 wherein m is an integer of 2 or 3, 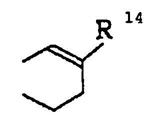 wherein R¹⁴ is a hydrogen atom or a C₁-C₄ alkyl group, 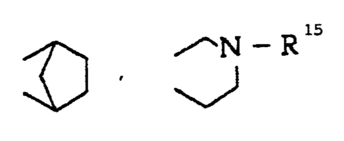 wherein R¹⁵ is a hydrogen atom or an aralkyl group, or 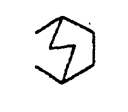 provided that when 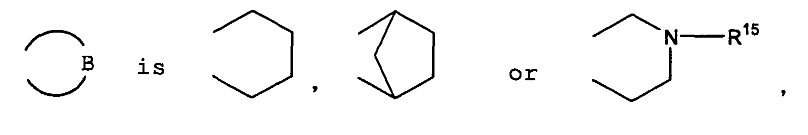 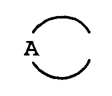 should not be 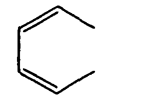 and R⁷ should not be a hydrogen atom, an enantiomer thereof, an acid addition salt thereof, or a hydrate or solvate thereof.

2. The therapeutic agent of claim 1, wherein the compound of the formula (I) is a compound of the formula (Ia) 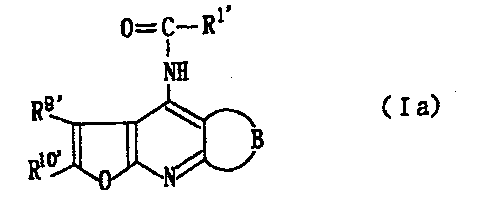 wherein R^{1'} is a C₂-C₆ alkyl group or the formula (II)' 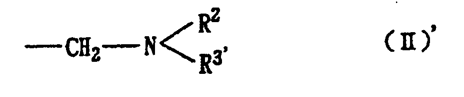 wherein R² is a hydrogen atom or an acetyl group, and R^{3'} is a C₁-C₆ alkyl group or 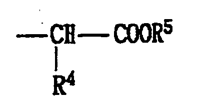 wherein R⁴ and R⁵ are each independently a hydrogen atom or a C₁-C₆ alkyl group, and in 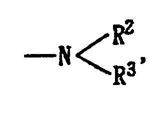 of the formula (II)', R² and R^{3'} may be linked to each other to form 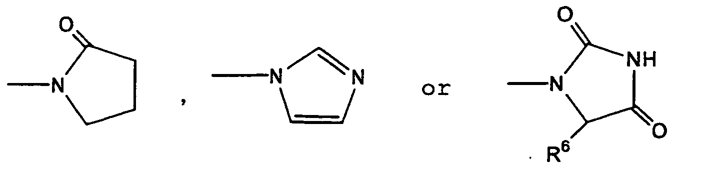 wherein R⁶ is a hydrogen atom or a C₁-C₆ alkyl group; R^{9'} and R^{10'} are each independently a C₁-C₄ alkyl group; and 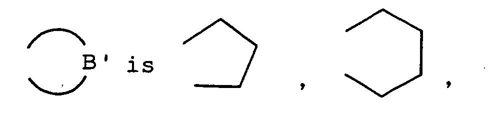 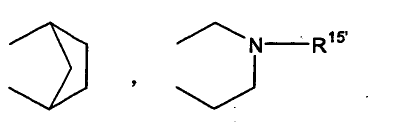 wherein R^{15'} is an aralkyl group, or

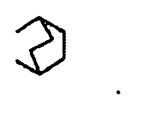

3. The therapeutic agent of claim 1, wherein the compound of the formula (I) is a 4-acylamino-5,6,7,8-tetrahydrofuro[2,3-b]quinoline derivative of the formula (Ib) 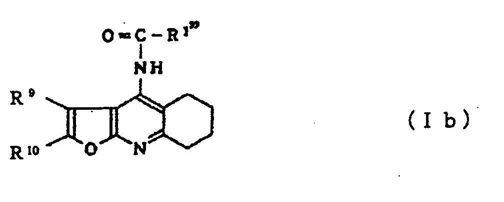 wherein R^{1"} is a C₂-C₆ alkyl group, 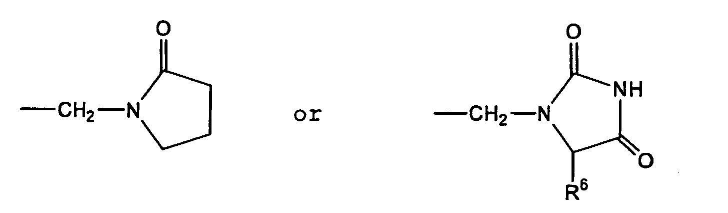 wherein R⁶ is a hydrogen atom or a C₁-C₆ alkyl group; and R⁹ and R¹⁰ are each independently a hydrogen atom or a C₁-C₄ alkyl group.

4. The therapeutic agent of claim 1, wherein the compound of the formula (I) is 2-(2-oxopyrrolidin-1-yl)-N-(2,3-dimethyl-5,6,7,8-tetrahydrofuro[2,3-b]quinolin-4-yl)acetamide.

5. The therapeutic agent of any of claims 1-4, wherein the condition of schizophrenia is a negative symptom or a cognitive disorder.
